# EUROPEAN PATENT APPLICATION

(11) **EP 2 735 265 A1**
(43) Date of publication of application: **28.05.2014**
(21) Application number: 12814145.4
(22) Date of filing: 18.07.2012
(51) Int. Cl.: A61B 5/00, A61B 5/11, A61B 5/16, A01K 1/03

(54) **DEVICE FOR ANIMAL EXPERIMENTATION IN NEUROSCIENCE RESEARCH**

(30) Priority: 19.07.2011 ES 201131227 P
(71) Applicant: Universidad Pablo de Olavide, 41013 Sevilla (ES); Universitat Autònoma De Barcelona, 08193 Bellaterra (Barcelona) (ES)
(72) Inventor: MARTÍN PASCUAL, Miguel Angel, E-08193 Bellaterra (Barcelona) (ES); ANDREU SÁNCHEZ, Celia, E-08193 Bellaterra (Barcelona) (ES); SANTOS, José Antonio, E-41013 Sevilla (ES); GRUART I MASSO, Agnés, E-41013 Sevilla (ES); DELGADO-GARCIA, José M., E-41013 Sevilla (ES)
(74) Representative: Temino Ceniceros, Ignacio
(86) International application number: PCT/ES2012/070544
(87) International publication number: WO 2013/011186

(57) **Abstract**

The invention relates to a device for animal experimentation in neuroscience research, formed by a prismatic frame (1) to which touch screens are secured for use as input/output devices of a computer system. The device also comprises a data acquisition system (2) and means (3) for processing said data. The device can be used to perform complex experiments in a fast and flexible manner owing to the experimentation software design. The device can be used to immerse a subject (7) being studied in a virtual reality environment.

## Description

The present device falls within the devices for the study of animal or human behavior. More specifically, it can be used to perform experiments that will be applied in the field of neuroscience, psychology, medicine and audiovisual reception studies and teaching and learning studies.

The present invention falls within the research into the behavior and the measuring and neuropsychological and neurophysiological research devices. It is also of interest to the computer industry and studies of brain-machine communication.

### State of the prior art

For the study of animal behavior have been used the so-called problem boxes in which a driving means such as for example a lever, was placed, which the animal being studied should actuate to achieve a concrete result, for example, the actuation of the lever to open the box and get out of it. The problem boxes are devices that are used to study the learning based on trial and error.

Subsequently, the use of the so-called Skinner boxes based on the problem box and used to analyze the behavior and learning of animals, was introduced. These boxes allow studying the operant conditioning.

Skinner box allows teaching to the trial subjects, to perform certain actions, such as pressing a lever to get food or drink. More modem designs of the former box allow the self-stimulation of the subject through electrodes or self-administration of substances, mediating cognitive or motor challenges. All of this may be caused previously or through the operation of mechanical-electrical levers by means of sounds, visual stimuli or similar that precede or follow rewards or punishments.

The usual structure of Skinner boxes includes a space that allows housing the specimen being studied so that it can perform various movements, actuate levers or, less occasionally, restrict their movements to achieve focused attention or certain postures. Different accessories release visual stimuli such as light spots, release auditory stimuli through speakers, or provide electric shock through a lower grid. Complementary accessories such as photoelectric cells, allow locating the animal in the box, or counting its approaches or handlings. Today, the entire system is connected to a computer system that quantifies or analyzes the events and their frequency.

The problem of Skinner boxes used in the State of the art is that they only allow an invariable design of the experiments. In addition the electromechanical connections required to perform studies with Skinner boxes are complex and they are designed for specific experiments.

The document WO-2011017328 describes a multi-screen device for vision testing and training. The invention provides a system and method for receiving an input by way of a touch-screen device. The touch-screen device displays a graphical element such that the information can be received by physical contact between the subject and the display surface of the touch-screen device. The touch-screen device communicates the input signal to the sensory training device utilizing a wireless communication protocol.

The document JP-62222105 describes an apparatus for analyzing the animal behavior without imparting stress to the subject being studied. It consists of a Skinner box capable of permitting the activity of the specimen and comprising a sensor, a device detecting the position of the specimen, and a system for processing the output from this device and recording the obtained data.

The document US-2010182247 proposes a device including one or more touch-sensitive display screens and software for use. It is configured to capture inputs through contact by the subject with the screen and, in the case of more than one screen, it allows displaying from the contact by the subject with one of the screens items to act on other screens.

The document CN-101584585 discloses a device and software used by it for the study of the behavior of white rats. Experiments are carried out in conditions of low noise and low illumination for no modification of the rat behavior.

The document CN-201393459 describes an animal intelligent training system, which comprises a training control unit, a display unit, an image monitoring unit and an animal activity box.

Other documents of the present invention that provide devices or software for the study of the animal behavior are CN-101011036, CN-1464466, WO-2010085645, CN-101715739, CN-201393459, CN-101021745, WO-2006040791 or US-2005065452.

### Explanation of the invention

The present invention aims to provide a device for neuroscience research that allows the presentation of interaction elements on touch screens in a fast and flexible manner.

In addition, the present invention proposes a device having manipulative touch or multitouch interaction of virtual objects, an adaptable immersive virtual multi-stimuli environment and activation capacity of the images displayed on screen from the cerebral electrical activity of experimental animal. This invention allows that animals can conceptually interact with a virtual learning interface for the first time.

The device of the present invention can be used to perform experiments more complex than those performed with devices known and used in the State of the art. In addition these experiments are configured in a fast and flexible manner. The change of presentation of stimuli or configuration of new experiments is carried out much more quickly and with economy of means. The presentation of touch interfaces and audiovisual information is carried out without mechanical handling.

Another advantage of this device is that less means are required to carry out the desired experiments. Analysis of new neuroscientist fields such as metacognition in animal learning is achieved due to the speed and flexibility with which experiments that previously were difficult to be constructed are configured with this device.

The present invention proposes a device for animal experimentation in neuroscience research comprising a prismatic frame, a data acquisition system, means for processing data and a computer system. The data acquisition system of the device is connected to a computer system in which the means for processing data are and that is connected to a plurality of touch screens. The touch screens are secured to the prismatic frame by way of walls and act as an input/output device of the computer system.

The touch screens are placed in the prismatic frame being secured to it through a mechanical fastening system allowing their easy interchangeability. This enables the touch screens can be replaced by others in the case if necessary maintenance and it allows its change by another type of screen. In addition one can have a hybrid device in which some of the sides of the prismatic frame are covered with touch screens and the rest of the sides are lined with walls of methacrylate, grids, etc. depending on the experiment to be carried out.

The touch screens used in the device of the invention can be either capacitive or resistive. The type of screen used depends on the area in which the screen is placed. Thus, if the screen is placed in a position in which the contact areas of the subject being studied with screen are for example hooves, nails, beaks or chitinous areas, the screens used are resistive screens. When the contact area of the subject being studied with the screen is for example plantar pad, hair, whiskers, etc., the screen used is a capacitive screen.

Each screen can have independent information or the screens can be coordinated by a computer system. The screens of the device can project a visual environment or display objects and interactive events, such as buttons, subscreens, animations, etc. In the event that the screens are connected to a computer system, each one of them communicates with the computer system by means of pulse signals. If separate screens are used, each of them incorporates its own computer system for data recording, event detection and presentation of environments.

The touch screens of the invention are used to display the environments desired in each experiment in addition to display items with which the subject object of study interacts. Thus, the screen is used as an input device to the computer system since it is used so that the subject being studied actuates levers, presses buttons, etc. that are displayed on the screen. The computer system collects these data and processes them with the means for processing data and emits an output that is displayed on the touch screens. The output emitted by the means for processing data is shown on the screens. Thus the device of the present invention allows changing the type of tests carried out to the subject depending on the actions it is doing. All this is carried out in a fast and flexible manner allowing a greater precision in the studies.

The data acquisition system used in the device of the invention is the type ADC (analog-to-digital converter) that captures electroencephalographic signals of the subject being studied, converts them to digital signals and sends them to the computer system.

Thus the output displayed on the screen is a function on the one hand of the input information which is obtained from the screen itself and the data obtained with the data acquisition system.

The software used varies depending on the particular experiment to be performed. This software can be activated from the electric activity of the subject being studied, for which an electroencephalogram is used or potentials evoked are used, or from their behavior and their motor activity. That is, the software utilized can be activated with signals coming to the computer system from the data acquisition system (that captures the electroencephalographic signal of the subject being studied) or from the input data that the screen sends to the computer system (the screen captures the activity of the subject being studied).

The touch screens utilized can have cameras. In this way the experiment can be recorded from different angles without using device does not belong to the experimental environment.

In another embodiment of the invention, the screens incorporate an infrared positioning system.

The device of the present invention can be connected with other devices that are necessary for the experiments such as for example a food dispenser device, a device for opening the door of one of the walls so that the subject being studied passes through it, etc.

Thus, the present invention allows studying the behavior of laboratory animals, or even humans if the scale is changed, in an immersive environment of virtual reality. When the device of the invention is used with humans, studies of perception, therapies and recreational applications of virtual games can be performed.

One of the most important advantages of the present invention is that it allows studying the direct cognitive relation of the animal by managing the presentation of objects in touch screens depending on the mental impulses of the subject under investigation.

Throughout the description and claims, the word "comprises" and its variations are not intended to exclude other technical features, additives, components or steps. For those skilled in the art, other objects, advantages and characteristics of the invention will emerge in part from the description and in part from the practice of the invention. The following examples and drawings are provided by way of illustration, and are not intended to be limiting of the present invention. Furthermore, the present invention covers all the possible combinations of particular and preferred embodiments herein indicated.

### Brief description of the drawings

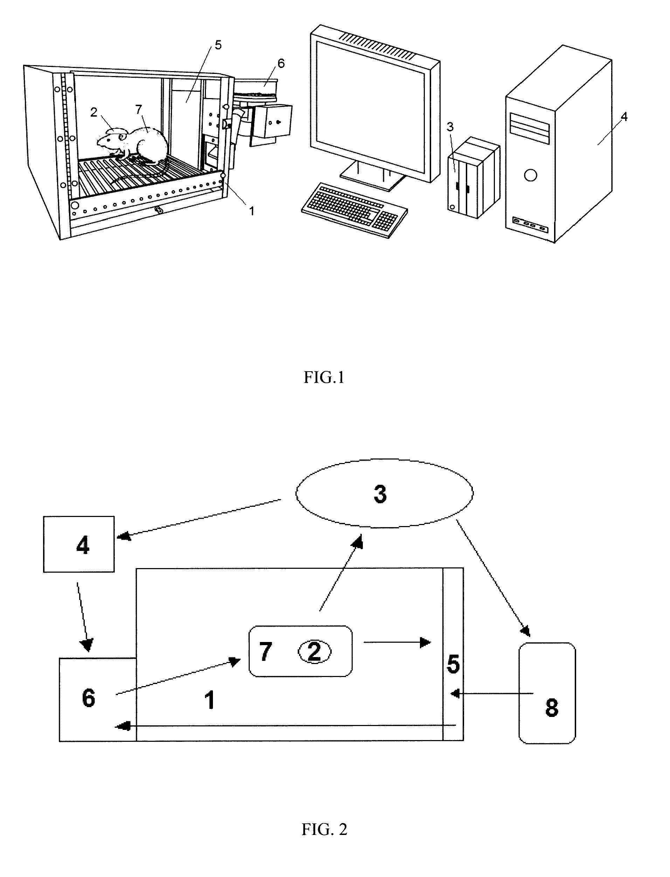

FIG. 1 shows a device of the present invention with a subject being studied on which the experiments designed for the device are carried out.
FIG. 2 shows a scheme of the operation of the invention.

### References:

1: Frame, 2: Data acquisition system; 3: Means for processing data; 4: Computer system; 5: Touch screen; 6. Food dispenser; 7: Subject being studied.

### Detailed explanation of embodiments

The device of the invention implements an algorithm the input of which can be the behavior of the subject (7) being studied or its own brain activity. To capture the brain activity of the subject being studied is used a commercial data acquisition system (2) ADC, i.e., an analog-to-digital converter. Electroencephalographic signals captured from the subject are analyzed in the internal unit incorporated in the device where the desired signals are selected and converted into digital signals to be processed in a processor.

The captured electroencephalographic signals are recorded online using conventional amplifiers. This can be made through cables or telemetry systems. The recorded signals can be stored in a logical or physical support for future analysis for recreation of these using a DAC system, i.e., digital-to-analog converter. In addition, the obtained statistical results are stored by the processor in a file. Saved data can be represented or analyzed with additional available programs.

The digital outputs of the algorithm will be used, through TTL pulses of programmable duration to actuate on a relay connected to a keyboard that simulates the pressing of a key, reaching this information to the processor. The activation of the mentioned key will activate the task in the program that is running.

This embodiment has used iPad touch screens (5) and the algorithm has been developed with Apple iOS. It also comprises a MP150 unit and a UIM100C unit. The MP150 unit is a high speed data acquisition system (2) that uses the latest technology in communication via networking. It is compatible with any UDP protocol both in Windows and in Macintosh. It allows the access to multiple MP150 systems in a local area, and saving the data of these units on a single computer connected to the local network. The system allows configuring the sampling frequency independently in each subsystem, being the maximum total rate of 400 kHz.

The research subjects interact in this device with touch screens (5) in a cognitive and manipulative manner, either by buttons, irregular areas, images, colors, virtual spaces, presented in touch screens (5) with dedicated software and carry out choices through this novel interface.

In an embodiment, software, that is an application installed on an iPad-type Tablet PC with a distribution system *ad-hoc* to be installable on different devices, has been used. The algorithm has been developed using Xcode4 for iOS and the tools used were Xcode 3, Xcode4, Interface Builder and Instruments.

The touch screens (5) utilized must be of the Tablet PC type. In this case Apple's iPad has been chosen. The used touch screens (5) have LED backlighting, 16 GB capacity, screen size: 768 x 1024 px, accelerometer, light sensor, digital compass, WiFi, bluetooth, stereo headphone jack, built-in speaker, microphone, 30-pin connector port for external hardware, on/off sleep/wake and screen rotation lock button.

The object of the software is to display visual stimuli that the subject (7) being studied, a rat, for example, be able to touch on the touch screen (5) of the used Tablet. In the present example, when touching the virtual button, the rat receives the food reward from a food dispenser (6) connected to the rest of the device. The software allows the rotation of the screen in portrait mode to optimize the positions of the virtual buttons.

The algorithm of this embodiment has different colors which in this case are white, black and blue, that are colors that are comprised in the chromatic range dimensioned for the color vision of the rat.

This algorithm allows combining different audiovisual stimuli, including video. The size of the stimuli can be changed when the rat interacts with them, to increase the complexity of their work and the position of stimuli can also be changed when the rat interacts.

The algorithm implemented in this example embodiment has been designed thinking in the rat as their recipient and user. However, it could be easily applied to other experimental animals.

Six buttons are displayed on the home screen of this example embodiment. Each selection leads to a simple experiment in which the recipient of the experiment can touch a stimulus, a button of specific, to achieve a response, for example, food. All buttons are on a gray background, in keeping with the light environment of the physical box and the laboratory. Both background and buttons are configurable in color according to the specific needs of each experiment.

The algorithm is designed to allow resizing the button, changing its position and the simultaneous change of size and position.

On the home screen is displayed an info button that shows the development of the application, the version and other criteria of use. The navigation over the application is fully touch-sensitive and intuitive. Every time we have entered in one of the described selections we can not return to the home screen. In order to access again to the home screen it is necessary to abandon the application and re-enter to prevent the recipient, the rat in this case, touches, voluntarily or involuntarily, said button and discontinues the experiment.

The algorithm of the invention is susceptible to develop also for other operating systems existing in other trademarks of touch tablets that are currently on the market such as Android, Symbian, WebOS or Windows 7, among others.

The example embodiment proposed utilizes a software that has a grey wallpaper of 40% imitating the luminosity and the walls of the usual research environments. This background can change in contrast, gamma and color according to specific requirements. In this case the subjects (7) being studied have been rats, therefore an application that showed a 260 x 104 pixels button in the visible blue spectrum for the limited color vision of employed rats has been used. Each press of the button by the rat is taken as a pulse for the experiment performed with this example. With software used in the example, spurious presses have been prevented.

Data acquisition is performed by an analog-to-digital converter that captures the animal encephalographic signals and converts them to digital signals. These signals are sent to the computer system that records them to have a historic record of the experiment results or to subsequently represent them or analyze them with additional programs especially designed for that purpose.

This particular example of the present invention uses a MP system. The system will comprise a MP150 unit and a UIM100C unit. The MP150 unit is a high speed data acquisition system (2) that uses a technology of communication via networking. This unit allows the access to multiple MP150 systems in a local area and saving the data of these units on a single computer connected to the local network. The computer system (4) used allows configuring the sampling frequency independently in the case of different subsystems.

## Claims

1. Device for animal experimentation in neuroscience research comprising a prismatic frame (1) in which the subject (7) being studied is introduced, a data acquisition system (2), means (3) for processing data and at least a computer system (4), and which is **characterized in that** the data acquisition system (2) is connected to the means (3) for processing data that are in the computer system (4) and said computer system (4) is connected to a plurality of touch screens (5), secured to the prismatic frame (1), that act together with other input/output elements of the device as an interface of the subject (7) being studied with the computer system (4).

2. Device according to claim 1, wherein the touch screens (5) are secured to the prismatic frame (1) through a mechanical fastening system allowing their interchangeability.

3. Device according to claim 1, wherein the data acquisition system (2) is an analog-to-digital converter that is connected with the computer system and that captures the encephalographic signals of the subject (7) being studied, converts them to digital signals and sends them to the computer system (4).

4. Device according to claim 1 implementing an algorithm that has a button in the touch screen (5) in a certain color and size and that allows changing the size, color, position and the different combinations of these variables depending on the experiment to be carried out.

5. Device according to claim 1 implementing an algorithm that allows the multi-touch presentation of images, auditory stimuli, and audiovisual forms in general.
